(19)

**Europäisches Patentamt**

**European Patent Office**

**Office européen des brevets**

(11) **EP 4 640 211 A1**

(12) **EUROPEAN PATENT APPLICATION**

(43) Date of publication:
**29.10.2025 Bulletin 2025/44**

(21) Application number: **24171976.4**

(22) Date of filing: **23.04.2024**

(51) International Patent Classification (IPC):
**A61K 9/107** (2006.01)  **A61K 9/19** (2006.01)
**A61K 31/00** (2006.01)  **A61K 47/28** (2006.01)
**A61P 3/02** (2006.01)  **A61K 9/00** (2006.01)

(52) Cooperative Patent Classification (CPC):
(C-Sets available)
**A61K 9/1075; A61K 9/19; A61K 31/07;**
**A61K 31/122; A61K 31/355; A61K 31/592;**
**A61K 31/593; A61K 47/28; A61P 3/02;**
A61K 9/0019                          (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **B. Braun Melsungen AG**
**34212 Melsungen (DE)**

(72) Inventors:
• SCHWEBEL, Herve
 **34212 Melsungen (DE)**
• ADAMO, Vincent
 **34212 Melsungen (DE)**

(74) Representative: **Maiwald GmbH**
**Elisenhof**
**Elisenstraße 3**
**80335 München (DE)**

(54) **PHARMACEUTICAL COMPOSITION COMPRISING WATER-INSOLUBLE VITAMIN AND MIXED MICELLES**

(57)    The present disclosure relates to a pharmaceutical composition comprising mixed micelles, and at least one water-insoluble vitamin, as well as to a method for manufacturing said pharmaceutical composition and the use thereof; the mixed micelles comprising at least one phospholipid and at least one pharmaceutically acceptable bile salt.

EP 4 640 211 A1

(52) Cooperative Patent Classification (CPC): (Cont.)

C-Sets
**A61K 31/07, A61K 2300/00;**
**A61K 31/122, A61K 2300/00;**
**A61K 31/355, A61K 2300/00;**
**A61K 31/592, A61K 2300/00;**
**A61K 31/593, A61K 2300/00**

## Description

### Field of the invention

The present disclosure relates to a pharmaceutical composition comprising mixed micelles, and at least one water-insoluble vitamin, as well as to a method for manufacturing said pharmaceutical composition and the use thereof; the mixed micelles comprising at least one phospholipid and at least one pharmaceutically acceptable bile salt.

### Background

Lecithin based mixed micelles have been used for the solubilization of poorly water-soluble (drug) substance(s). These mixed micelles may be composed of phospholipids and one or more surfactants which are able to disrupt and solubilize phospholipid bilayers in a certain ratio of surfactant to phospholipid.
Phospholipids can be obtained from natural source (plant or animal as sources, such as, e.g., soy bean lecithin or egg lecithin) or by chemical synthesis. Surfactants used for mixed micelles are typically glycocholic acid, macrogol 15 hydroxystearate, polysorbate 80 or polyoxyl 35 castor oil.
The preparation of mixed micelles is conventionally performed via a film method, comprising the dissolution of all (lipophilic and amphiphilic) components, lecithin, surfactant(s) and (drug) substance(s) in a volatile organic solvent, such as alcohols or chlorinated solvents, and the subsequent removal of the solvent to obtain a film in which the drug substance is intimately incorporated in a matrix of mixed micelles. Said film method is typically performed in lab scale via glass round-bottom flasks in a rotation evaporator; in larger scale, spray drying has been established for drying the lecithin/surfactant mixture.
Said method is widely used for the preparation of vitamin containing products, in particular as lyophilized products. These products may then be reconstituted for use, e.g., in parenteral nutrition.
During the above given process, a film of mixed micelles comprising the poorly water-soluble (drug) substance(s) is obtained as intermediate material. However, said intermediate material has limited stability and shelf-life, requiring further steps for stabilization or complex logistical supply.
Furthermore, the above given process requires the use and eventually the removal of a solvent. This can be achieved by means of various well established process technologies, such as freeze drying, spray drying, oven drying. However, due to the presence of volatile organic solvents, handling and drying requires costly explosion proof installations. In addition, residual organic solvent levels have to be kept at a minimum to ensure compliance to current pharmaceutical guidelines. Finally, the use of organic solvents should be avoided to reduce environmental impact.

### Summary

There is thus still a need for a pharmaceutical composition comprising water-insoluble vitamins which is highly stable, but which does not contain any organic solvents and low amounts of pharmaceutically non-active excipients, such as phospholipids and bile salt. Furthermore, there is still a need for a method of manufacture of such pharmaceutical composition which method does not make use of organic solvents.
In a first aspect, the present disclosure relates to a pharmaceutical composition comprising mixed micelles, and at least one water-insoluble vitamin selected from the group consisting of vitamin A, vitamin D, vitamin E, and vitamin K, and pharmaceutically acceptable derivatives thereof,

a) wherein the mixed micelles each comprise at least one phospholipid, and at least one pharmaceutically acceptable bile salt, and
b) wherein at least one of the following conditions is met:

i) ratio of vitamin A (total amount in IU) to mixed micelles (total amount in mg) is more than 15 IU/mg, or
ii) ratio of vitamin D (total amount in IU) to mixed micelles (total amount in mg) is more than 0.9 IU/mg, or
iii) ratio of vitamin E (total amount in IU) to mixed micelles (total amount in mg) is more than $45 \times 10^{-3}$ IU/mg, or
iv) ratio of vitamin K (total amount in mg) to mixed micelles (total amount in mg) is more than $0.6 \times 10^{-3}$.

In a second aspect, the present disclosure relates to method for manufacturing the pharmaceutical composition according to the first aspect comprising the steps of

a) providing a dispersion of at least one phospholipid in water (mixture I);
b) adding a pharmaceutically acceptable bile salt under mixing to mixture I to obtain a second mixture (mixture II);
c) adding the at least one water-insoluble vitamin selected from the group consisting of vitamin A, vitamin D, vitamin E, and vitamin K, and pharmaceutically acceptable derivatives thereof, to mixture II under mixing;
d) optionally adding a further amount of mixture II, wherein the ratio of phospholipid to bile salt is identical or different to the mixture prepared in step b;

to provide the pharmaceutical composition.

In a third aspect, the present disclosure relates to the pharmaceutical composition according to the first aspect for use in a method of treating or preventing a vitamin deficiency in a subject.

## Detailed Description

In a first aspect, the present disclosure relates to a pharmaceutical composition comprising mixed micelles, and at least one water-insoluble vitamin selected from the group consisting of vitamin A, vitamin D, vitamin E, and vitamin K, and pharmaceutically acceptable derivatives thereof,

a) wherein the mixed micelles each comprise at least one phospholipid, and at least one pharmaceutically acceptable bile salt, and

b) wherein at least one of the following conditions is met:

i) ratio of vitamin A (total amount in IU) to mixed micelles (total amount in mg) is more than 15 IU/mg, or

ii) ratio of vitamin D (total amount in IU) to mixed micelles (total amount in mg) is more than 0.9 IU/mg, or

iii) ratio of vitamin E (total amount in IU) to mixed micelles (total amount in mg) is more than $45 \times 10^{-3}$ IU/mg, or

iv) ratio of vitamin K (total amount in mg) to mixed micelles (total amount in mg) is more than $0.6 \times 10^{-3}$.

It was surprisingly found that, by using the below described method, a pharmaceutical composition may be prepared with a high ratio of water-insoluble vitamin to mixed micelles. This unusually high ratio of water-insoluble vitamin to mixed micelle allows for the preparation of a pharmaceutical composition with a high concentration of water-insoluble vitamins compared to other ingredients, in particular excipients, such as phospholipids and bile salt. These excipients are needed for the formulation of a storage-stable pharmaceutical composition, but they do not add to the therapeutic effect of the pharmaceutical composition. Accordingly, there is a desire to reduce the amount of excipients in a pharmaceutical composition, or, in other words, increase the ratio of active agent, such as water-insoluble vitamin, to excipient, such as phospholipid or bile salt. The pharmaceutical composition of the present disclosure provides for a high ratio of water-insoluble vitamin to excipient in the form of mixed micelles comprising at least one phospholipid and at least one bile salt.

By reducing the amount of excipients, in particular the amount of bile salt, a possibly given toxicity and side effects of the glycocholate may be reduced. Additionally, the pharmaceutical composition may be prepared at lower costs since less material is needed, and the pharmaceutical composition may also be freeze-dried easier because less material has to be freeze-dried.

### Mixed Micelles

The pharmaceutical composition of the present disclosure comprises mixed micelles. Each of the mixed micelles comprise at least one phospholipid and at least one pharmaceutically acceptable bile salt.

As used herein, the term "mixed micelle" refers to a mixture of at least one phospholipid and at least one pharmaceutically acceptable bile salt that may form a mixed micelle. In general, a micelle is formed by an amphiphilic molecule in a solvent. Preferably, the solvent is water, and the amphiphilic molecule is a surfactant.

In the context of the present disclosure, the mixed micelle is formed of at least one phospholipid and at least one pharmaceutically acceptable bile salt in water. A corresponding process preferably comprises dispersing at least one phospholipid in water until a homogeneous dispersion is obtained, followed by the addition of at least one pharmaceutically acceptable bile salt to obtain a clear formulation containing mixed micelles. The dispersion of the mixed micelles in water may be subjected to a drying step, such as a lyophilization, resulting in the "dried form of mixed micelles", which may also be referred to as precursors of mixed micelles. Such precursors of mixed micelles can be reconstituted with or redispersed in water to form a dispersion of mixed micelles in water. The present disclosure thus, when referring to a mixed micelle, refers to a form of a mixture of at least one phospholipid and at least one pharmaceutically acceptable bile salt that is present in an aqueous medium as mixed micelle, or that may form a mixed micelle when brought into contact with water. The "mixed micelle" may thus also be referred to as "mixed micelle or precursor thereof".

It should be noted in this context that the mere mixing of at least one phospholipid and at least one pharmaceutically acceptable bile salt in water will not form a dispersion of mixed micelles, in particular if a mixture of at least one phospholipid and at least one pharmaceutically acceptable bile salt with a lipophilic vitamin (such as any of the vitamins A, D, E and K) is brought into contact with water.

Phospholipid

For the preparation of the mixed micelles, in a first step a), a phospholipid is dispersed in water, preparing a first mixture (mixture I).

The at least one phospholipid may preferably be a lecithin, further preferably phosphatidylcholine, and more preferably a purified phosphatidylcholine. The phospholipid may be a soy bean lecithin or egg lecithin, i.e., a lecithin derived from soy bean or egg. A particularly preferred embodiment of a phosphatidylcholine is Lipoid S 100®.

The phospholipid preferably has a purity of at least 90 wt.-%, preferably of at least 95 wt.-%, more preferably at least 98 wt.-%, more preferably at least 99 wt.-%, more preferably at least 99.5 wt.-%, more preferably at least 99.9 wt.-%, most preferably in a pure form.

If a phosphatidylcholine is used as phospholipid, said phosphatidylcholine preferably has a purity of at least 90 wt.-%, preferably of at least 92 wt.-%, more preferably at least 94 wt.-%, more preferably at least 98 wt.-%, more preferably at least 99 wt.-%, more preferably at least 99.5 wt.-%, most preferably in a pure form.

In a preferred embodiment, the phospholipid as used in the preparation of mixture I is used in an amount of from about 10 to about 50 wt.% based on the total weight of mixture I, preferably of from about 15 to about 45 wt.% based on the total weight of mixture I, more preferably of from about 17 to about 40 wt.% based on the total weight of mixture I, more preferably of from about 20 to about 32 wt.% based on the total weight of mixture I, more preferably of from about 22 to about 31 wt.% based on the total weight of mixture I.

The preparation of mixture I is followed by the preparation of a formulation containing mixed micelles of phospholipid and a pharmaceutically acceptable bile salt in a second mixture (mixture II).

Pharmaceutically acceptable bile salt

After preparation of a dispersion of at least one phospholipid in water (mixture I), a formulation of mixed micelles containing a phospholipid and a pharmaceutically acceptable bile salt in water is prepared as a second mixture (mixture II) in step b.

In accordance with the present disclosure, any salt of a bile acid may be used, as long as it is pharmaceutically acceptable.

In a preferred embodiment, the pharmaceutically acceptable bile salt is a pharmaceutically acceptable salt of glycocholate or a pharmaceutically acceptable salt of taurocholate or a pharmaceutically acceptable salt of deoxycholate, preferably wherein the pharmaceutically acceptable bile salt is sodium glycocholate.

The pharmaceutically acceptable bile salt is added to mixture I to result in mixture II, comprising mixed micelles of at least one phospholipid and a pharmaceutically acceptable bile salt in water. The bile salt is acting as amphiphilic surfactant in the formation of mixed micelles together with the phospholipid.

The pharmaceutically acceptable bile salt preferably has a purity of at least 90 wt.-%, preferably of at least 95 wt.-%, more preferably at least 98 wt.-%, more preferably at least 99 wt.-%, more preferably at least 99.5 wt.-%, more preferably at least 99.9 wt.-%, most preferably in a pure form.

In a preferred embodiment, mixture II comprises the pharmaceutically acceptable bile salt in an amount of from about 5 to about 50 wt.% based on the total weight of mixture II, preferably of from about 15 to about 45 wt.% based on the total weight of mixture II, more preferably of from about 20 to about 40 wt.% based on the total weight of mixture II, more preferably of from about 25 to about 40 wt.% based on the total weight of mixture II, more preferably of from about 27 to about 36 wt.% based on the total weight of mixture II.

In mixture II, mixed micelles of at least one phospholipid and a pharmaceutically acceptable bile salt are present in an aqueous formulation. The bile salt is added to the previously prepared mixture of phospholipid and water (mixture I), and the above given amounts of pharmaceutically acceptable bile salt are relative to the total amount of mixture I, i.e., the dispersion of phospholipid in water.

Mixture containing mixed micelles

In the mixture comprising at least one phospholipid and at least one pharmaceutically acceptable bile salt (mixture II), a specific ratio of phospholipid to pharmaceutically acceptable bile salt in mixture II is present. Said ratio is preferably given as molar ratio, calculated on the respective molar weight of the phospholipid and the pharmaceutically acceptable bile salt.

In a preferred embodiment, mixture II comprises the at least one phospholipid and the pharmaceutically acceptable bile salt in a ratio of bile salt : phospholipid in the range of from about 7.5 : 1 (mol/mol) to about 1.5 : 1 (mol/mol); preferably from about 7 : 1 (mol/mol) to about 1.5 : 1 (mol/mol), more preferably of from about 6 : 1 (mol/mol) to about 1.6 : 1 (mol/mol), more preferably of from about 5 : 1 (mol/mol) to about 1.7 : 1 (mol/mol), and more preferably of from about 3 : 1 (mol/mol) to about 1.8 : 1 (mol/mol). Adjusting the ratio of bile salt: phospholipid in the mixed micelles in mixture II prior to addition of a liposoluble vitamin helps in forming a clear and homogenous formulation comprising mixed micelles and vitamins, i.e., mixture III.

After the addition of the pharmaceutically acceptable bile salt, mixture II contains mixed micelles of phospholipid and bile salt, dispersed in water. Said mixture II is the basic mixture for the addition of at least one water-insoluble vitamin. After addition of the at least one water-insoluble vitamin to mixture II, a resulting mixture III is obtained. Further amounts of a mixture II (containing mixed micelles of phospholipid and bile salt) may be added to mixture III. By adding further amounts of mixture II, the resulting composition is stabilized, allowing for the adjustment of the ratio of vitamin to mixed micelle, which may be used to optimize the final pharmaceutical composition for freeze-drying and storing. It is also possible to prepare further mixture containing mixed micelles at a different ratio of bile salt : phospholipid, thereby adjusting the ratio of bile salt : phospholipid in the final pharmaceutical composition. Also this measure may add to the stability of the final pharmaceutical composition.

The concentration of micelles comprising phospholipid and pharmaceutically acceptable bile salt, also referred to as mixed micelles, in mixture II is preferably above 40 wt.%, further preferably above 45 wt.%, further preferably above 48 wt.%, further preferably above 50 wt.%. Such concentration is helpful when adding the water-insoluble vitamin to mixture II and reduces the risk of disintegration of the mixed micelles in the process of adding the water-insoluble vitamin. The concentration of micelles comprising phospholipid and pharmaceutically acceptable bile salt is calculated as the total amount of phospholipid and pharmaceutically acceptable bile salt in relation to the total amount of mixture II.

In any aspect of the present disclosure, the water used is demineralized or distilled water of pharmaceutical grade.

### Water-insoluble vitamin

In the pharmaceutical composition of the present disclosure, at least one water-insoluble vitamin is comprised. Said at least one water-insoluble vitamin is also termed as liposoluble vitamin. The liposoluble vitamin is added to mixture II in a further step c), and then mixed to result in mixture III. Said resulting mixture III is a homogenous and clear formulation. In other words, the initial dispersion of the phospholipid in water is a homogenous and clear formulation at least after addition of a bile salt (resulting in mixture II) and the at least one water-insoluble vitamin (resulting in mixture III).

It is understood that in the pharmaceutical composition, at least one water-insoluble vitamin is comprised. This includes the use of one or more water-insoluble vitamins, such as 1, 2, 3, 4, 5, 6, 7, 8, 9, or 10, or more vitamins. Accordingly, also mixtures of water-insoluble vitamins may be used.

If more than one water-insoluble vitamin is used, the water-insoluble vitamins may be added to mixture II each separately or as a mixture of vitamins, including several mixtures of vitamins. The vitamins may then be added simultaneously or subsequently.

The at least one water-insoluble vitamin used in the present disclosure is thus part of the finally prepared pharmaceutical composition. Without being bound by any theory, it is assumed that the water-insoluble vitamin is encapsulated in the mixed micelle, i.e., it is comprised in the mixed micelle.

The water-insoluble vitamin may be any vitamin that is hardly, poorly, only very slightly soluble, practically insoluble or not soluble, i.e., insoluble, in water. For the present disclosure, a compound is considered water-insoluble if the solubility of the compound in water ($H_2O$ at 20 °C and pH of 7) is below 100 mg per 100 mL of water, or below 10 mg per 100 mL of water, or below 1 mg per 100 mL of water, or below 0.1 mg per 100 mL of water, or below 10 $\mu$g per 100 mL of water, or below 1 $\mu$g per 100 mL of water.

The at least one water-insoluble vitamin is selected from the group consisting of vitamin A, vitamin D, vitamin E, vitamin K, and pharmaceutically acceptable derivatives thereof. It is understood that in the context of the present disclosure, when reference to any of the vitamins A, D, E, or K is made, also derivatives thereof are comprised.

It is further preferred that the at least one water-soluble vitamin is a mixture of at least two, or at least three, or all members of the group consisting of vitamin A, vitamin D, vitamin E, and vitamin K, and pharmaceutically acceptable derivatives thereof.

In another preferred embodiment, the at least one water-insoluble vitamin comprises vitamin A, preferably in the form of vitamin A palmitate, and vitamin E, and optionally one or more further water-insoluble vitamins, preferably vitamin K and/or vitamin D.

In another preferred embodiment, the at least one water-insoluble vitamin comprises vitamin A, vitamin D, vitamin E, and vitamin K. In a further preferred embodiment, the at least one water-insoluble vitamin comprises vitamin A palmitate, vitamin D, vitamin E, and vitamin K.

In another preferred embodiment, the at least one water-insoluble vitamin has a purity of at least 95 wt.-%, preferably at least 98 wt.-%, more preferably at least 99 wt.-%, more preferably at least 99.5 wt.-%, more preferably at least 99.9 wt.-%, most preferably in a pure form. In other words, the at least one water-insoluble vitamin is added as raw material, such as in solid form, i.e., not as a solution.

Vitamin A

Vitamin A as used in the present disclosure refers to a group of organic compounds that includes retinol, retinal (also known as retinaldehyde), retinoic acid, and several provitamin A carotenoids (most notably beta-carotene [$\beta$-carotene]), and derivatives thereof. Any one of these compounds, or any mixture thereof, may be used when reference is made to vitamin A.

The pharmaceutical composition may comprise in a single unit, i.e., in a single container, from 2950 to 3650 IU, preferably from 3100 to 3500 IU, more preferably about 3300 IU vitamin A. Vitamin A can be used in different forms, in particular different derivatives. For example, vitamin A can be used in the form of retinol, retinyl esters, retinyl palmitate, retinyl acetate, or other derivatives of vitamin A. In a particularly preferred embodiment, retinyl palmitate is used as vitamin A.

The water-insoluble vitamin A, if present in the pharmaceutical composition, is dissolved or comprised in mixed micelles as defined above.

In a preferred embodiment of the first aspect of the present disclosure, the pharmaceutical composition comprises vitamin A, i.e., the at least one water-insoluble vitamin comprises vitamin A, and the ratio of vitamin A (total amount in IU) to mixed micelles (total amount in mg) is 20 IU/mg or more, preferably 25 IU/mg or more, more preferably 30 IU/mg or more, yet more preferably 40 IU/mg or more, even more preferably 50 IU/mg or more; and/or 100 IU/mg or less, preferably 90 IU/mg or less, more preferably 75 IU/mg or less, yet more preferably 60 IU/mg or less, yet more preferably 50 IU/mg or less, even more preferably 40 IU/mg or less.

Vitamin D

Vitamin D as used in the present disclosure refers to the group of fat-soluble secosteroids as known to the skilled person, comprising vitamin D1 to D5. Any one of these vitamins D1 to D5, or any mixture, may be used when reference is made to vitamin D.

The pharmaceutical composition may comprise in a single unit, i.e., in a single container from 180 to 220 IU (corresponding to 4.5 to 5.5 $\mu$g), preferably from 192 to 212 IU (corresponding to 4.8-5.3 $\mu$g), more preferably about 200 IU (corresponding to 5.0 $\mu$g) vitamin D (preferably vitamin D3). The term "vitamin D" as used herein refers to "vitamin D2" (ergocalciferol) and/or "vitamin D3" (cholecalciferol). Preferably, vitamin D3 is used. Vitamin D3 can be used in the form of cholecalciferol or derivatives thereof. If not specified otherwise, the amounts given herein for vitamin D, preferably vitamin D3, refer to cholecalciferol. A person skilled in the art is aware that when vitamin D3 is used in a form other than cholecalciferol, the amounts need to be adapted (based on the molecular weight). Particularly preferably, vitamin D3 is used in the form of cholecalciferol.

The water-insoluble vitamin D, if present in the pharmaceutical composition, is dissolved or comprised in mixed micelles as defined above.

In a preferred embodiment of the first aspect of the present disclosure, the pharmaceutical composition comprises vitamin D, i.e., the at least one water-insoluble vitamin comprises vitamin D, and the ratio of vitamin D (total amount in IU) to mixed micelles (total amount in mg) is 0.95 IU/mg or more, preferably 1.0 IU/mg or more, more preferably 1.3 IU/mg or more, yet more preferably 1.5 IU/mg or more, even more preferably 2.0 IU/mg or more; and/or 10 IU/mg or less, preferably 8 IU/mg or less, more preferably 6 IU/mg or less, yet more preferably 5 IU/mg or less, yet more preferably 4 IU/mg or less, even more preferably 3 IU/mg or less.

Vitamin E

Vitamin E as used in the present disclosure refers to the group of eight fat soluble compounds that include four tocopherols and four tocotrienols. Any of these eight compounds, or any mixture thereof, may be used. The use of alpha-tocopherol is preferred in connection with vitamin E.

The pharmaceutical composition may comprise in a single unit, i.e., in a single container, from 9 to 11 IU (corresponding to 8.2 to 10.0 mg), preferably from 9.5 to 10.5 IU (corresponding to 8.56 to 9.65 mg), more preferably about 10 IU (corresponding to 9.1 mg) vitamin E. Vitamin E can be used in different forms. For example, vitamin E can be used in the form of all-rac-$\alpha$-tocopherol (D/L-$\alpha$-tocopherol), all-rac-$\alpha$-tocopherol acetate, (*RRR*)-$\alpha$-tocopherol (D-$\alpha$-tocopherol), or other derivatives of vitamin E. If not specified otherwise, the amounts given herein for vitamin E refer to all-rac-$\alpha$-tocopherol. A person skilled in the art is aware that when vitamin E is used in a form other than all-rac-$\alpha$-tocopherol, the

amounts need to be adapted (based on the molecular weight). Particularly preferably, vitamin E is used in the form of all-rac-$\alpha$-tocopherol.

The water-insoluble vitamin E, if present in the pharmaceutical composition, is dissolved or comprised in mixed micelles as defined above.

In a preferred embodiment of the first aspect of the present disclosure, the pharmaceutical composition comprises vitamin E, i.e., the at least one water-insoluble vitamin comprises vitamin E, and the ratio of vitamin E (total amount in IU) to mixed micelles (total amount in mg) is $50\times10^{-3}$ IU/mg or more, preferably $60\times10^{-3}$ IU/mg or more, more preferably $80\times10^{-3}$ IU/mg or more, yet more preferably $90\times10^{-3}$ IU/mg or more, even more preferably $100\times10^{-3}$ IU/mg or more; and/or $400\times10^{-3}$ IU/mg or less, preferably, preferably $350\times10^{-3}$ IU/mg or less, more preferably $300\times10^{-3}$ IU/mg or less, yet more preferably $250\times10^{-3}$ IU/mg or less, yet more preferably $200\times10^{-3}$ IU/mg or less, even more preferably $150\times10^{-3}$ IU/mg or less.

Vitamin K

Vitamin K as used in the present disclosure refers to a group of structurally similar fat-soluble vitamins, comprising vitamin K1, vitamin K2, and vitamin K3. Any of these vitamins, or any combination thereof, may be used. However, it is preferred to use vitamin K1, and/or vitamin K2, further preferably vitamin K1.

The pharmaceutical composition may comprise in a single unit, i.e., in a single container, from 135 to 165 $\mu$g, preferably from 143 to 158 $\mu$g, more preferably about 150 $\mu$g vitamin K1. Vitamin K1 can be used in different forms. For example, vitamin K1 can be used in the form of phytomenadione or other derivatives of vitamin K1. If not specified otherwise, the amounts given herein for vitamin K1 refer to phytomenadione. A person skilled in the art is aware that when vitamin K1 is used in a form other than phytomenadione, the amounts need to be adapted (based on the molecular weight). Particularly preferably, vitamin K1 is used in the form of phytomenadione.

The water-insoluble vitamin K, if present in the pharmaceutical composition, is dissolved or comprised in mixed micelles as defined above.

In a preferred embodiment of the first aspect of the present disclosure, the pharmaceutical composition comprises vitamin K, i.e., the at least one water-insoluble vitamin comprises vitamin K, and the ratio of vitamin K (total amount in mg) to mixed micelles (total amount in mg) is $0.65\times10^{-3}$ or more, preferably $0.7\times10^{-3}$ or more, more preferably $0.75\times10^{-3}$ or more, yet more preferably $1.0\times10^{-3}$ or more, even more preferably $1.3\times10^{-3}$ or more; and/or $10\times10^{-3}$ or less, preferably $6\times10^{-3}$ or less, more preferably $5\times10^{-3}$ or less, yet more preferably $4\times10^{-3}$ or less, yet more preferably $3\times10^{-3}$ or less, even more preferably $2\times10^{-3}$ or less.

***Water-soluble vitamins***

The pharmaceutical composition of the present disclosure may additionally comprise at least one water-soluble vitamin. Said at least one water-soluble vitamin is provided after the at least one water soluble vitamin is added to the mixture of mixed micelles and water, and it forms a homogenous and clear formulation.

The pharmaceutical composition of the present disclosure may contain one or more water-soluble vitamins. This includes the use of one or more water-soluble vitamins, such as 1, 2, 3, 4, 5, 6, 7, 8, 9, or 10, or more water-soluble vitamins. Accordingly, also mixtures of water-soluble vitamins may be used.

The at least one water-soluble vitamin is added to the mixture containing mixed micelles as solution. In a preferred embodiment, the solution is an aqueous solution, i.e., the at least one water-soluble vitamin is provided and added to the mixture containing mixed micelles as solution in water. In another preferred embodiment, the at least one water-soluble vitamin is provided as more than one solution, i.e., as 2, 3, 4, 5, or more solutions. These solutions may comprise different water-soluble vitamins, or the same water-soluble vitamins in different or identical concentrations.

The at least one water-soluble vitamin used in the present disclosure is thus part of the finally prepared pharmaceutical composition. The water-soluble vitamin may be any vitamin that is easily water soluble. For the present disclosure, a compound is considered water-soluble if the solubility of the compound in water ($H_2O$ at 20 °C and pH of 7) is above 1 mg per 100 mL of water, or above 10 mg per 100 mL of water, or above 0.1 g per 100 mL of water, or above 0.5 g per 100 mL of water, or above 1 g per 100 mL of water.

The pH of the solution may be adapted according to the needs to dissolve the at least one water-soluble vitamin more easily.

In another preferred embodiment, the at least one water-soluble vitamin has a purity of at least 95 wt.-%, preferably at least 98 wt.-%, more preferably at least 99 wt.-%, more preferably at least 99.5 wt.-%, more preferably at least 99.9 wt.-%, most preferably in a pure form.

In another preferred embodiment, the water-soluble compound is the water-soluble form of a vitamin, such as a water-soluble salt, preferably selected from the group consisting of vitamin C, vitamin B1 (thiamine), vitamin B2 (riboflavin), vitamin B6 (pyridoxine), Vitamin B12 (cobalamin), vitamin B9 (folate), vitamin B5 (pantothenic acid), vitamin B7 (biotin), vitamin B3 (niacin), and mixtures thereof.

It is further preferred that the water-soluble vitamin is a mixture of at least two, or at least three, or at least four, or at least five, or at least six, or at least seven, or at least eight, or all members of the group consisting of vitamin C, vitamin B1, vitamin B2, vitamin B6, vitamin B12, vitamin B9, vitamin B5, vitamin B7, and vitamin B3.

It may further be preferred to add the vitamins in two separate solutions to the mixture containing mixed micelles. Accordingly, a first solution containing vitamins is prepared, wherein the first solution contains at least one vitamin selected from the group consisting of vitamin C, vitamin B1, vitamin B2, vitamin B3, vitamin B5, and vitamin B6; and a second solution is, wherein the second solution contains at least one vitamin selected from vitamin B7, vitamin B9, and vitamin B12; wherein both solutions are aqueous solutions. It is further preferred that the first and second solutions contain all elements of the respective group. The first and second solution may be added in any order in the preparation of the pharmaceutical composition to the mixture containing mixed micelles and the at least one water-insoluble vitamin.

### *Pharmaceutical composition and use*

In a third aspect, the present disclosure relates to the pharmaceutical composition according to the first aspect for use in a method of treating or preventing a vitamin deficiency in a subject.

The pharmaceutical composition of the present disclosure may thus be used in a method of treating or preventing a vitamin deficiency in a subject.

The pharmaceutical composition is preferably free of any organic solvents and/or oils, i.e., the pharmaceutical composition does not contain an organic solvent and/or an oil. In a preferred embodiment, the organic solvent is selected from the group consisting of ethanol, methanol, chloroform, *tert*-butanol, glycerol, and *iso*-propanol. In particular, the pharmaceutical composition is preferably free of ethanol and/or methanol. It is therefore preferred that none of the mixtures used in the preparation of the pharmaceutical composition contain any organic solvent, such as ethanol and/or methanol, and no organic solvent is added in any step during the preparation method.

In another preferred embodiment, none of the method steps of the present disclosure comprises the addition of an organic solvent, such as ethanol and/or methanol. Even though no organic solvents are used in accordance with the method of the present disclosure, the resulting pharmaceutical composition may nevertheless contain residual amounts of organic solvent, such as ethanol or methanol, as inevitable impurities, if such solvents were used in the preparation of the starting materials or ingredients used in the method of the present disclosure.

Also, it is preferred that the pharmaceutical composition does not contain any oil, in particular any oil selected from the group consisting of soybean oil, olive oil, fish oil, fish oil extract, cottonseed oil, safflower oil, corn oil, sunflower oil, coconut oil, palm kernel oil, rapeseed oil, medium chain triglycerides, and mixtures thereof.

### *Dry pharmaceutical composition*

The pharmaceutical composition of the present disclosure may further be dried. This may increase stability of the pharmaceutical composition for increased shelf-life and storage times. In a preferred embodiment, the method for the preparation of a pharmaceutical composition comprises a drying step, resulting in a dry pharmaceutical composition. In a further preferred embodiment, the drying step comprises spray-drying, oven drying and/or lyophilization. The drying may be achieved by any known method, including spray-drying, oven drying and lyophilization (freeze-drying). It is preferred that the dry pharmaceutical composition is prepared by lyophilization.

In a preferred embodiment, the dry pharmaceutical composition has a residual water content of less than 2 wt.%, preferably less than 1 wt.%, preferably less than 0.5 wt.%, based on the total weight of the dry pharmaceutical composition.

The dry pharmaceutical composition may be re-constituted, e.g., in water, prior to administration to a patient.

In a preferred embodiment, the dry pharmaceutical composition may be reconstituted. When reconstituting, the dry pharmaceutical composition is mixed with water, preferably water for injection, and a dispersion of the mixed micelles is prepared by mixing, such as shaking or stirring. The application of ultrasonic waves may assist the reconstitution of the pharmaceutical composition.

The reconstituted pharmaceutical composition is preferably stable at least for 24 h after reconstitution in an injectable solution, determined by turbitdity and particle size, measured by nephelometry, with counts of 12 NTU or less, preferably 5 NTU or less, more preferably 3; and/or by dynamic light scattering (DLS), with particles sizes of 25 nm or less, preferably 20 nm or less, more preferably 15 nm or less, yet more preferably 10 nm or less and most preferably 8 nm or less. The stability of the reconstituted pharmaceutical composition may thus be measured by Nephelometry and/or DLS, and if the pharmaceutical composition fulfils either of the two criteria, preferably both, after 24 h or more, the pharmaceutical composition is considered stable. In other words, e.g., if the counts measure by nephelometry is at 12 NTU or less at least 24 h after reconstitution, the pharmaceutical composition is stable. Similarly, e.g., if the maximum particle size as measured by DLS is 25 nm or less, the pharmaceutical composition is stable.

A pharmaceutical composition according to the present disclosure is considered stable for at least 12 months when stored at 30 °C or less, or at 25 °C or less, or at 10 °C or less, or at 5 °C or less, if the pharmaceutical composition still fulfils the above given stability requirements for a reconstituted pharmaceutical composition. The stable pharmaceutical composition is preferably a dry pharmaceutical composition.

**Method of manufacturing**

In a second aspect, the present disclosure relates to method for manufacturing the pharmaceutical composition according to the first aspect comprising the steps of

a) providing a dispersion of at least one phospholipid in water (mixture I);
b) adding a pharmaceutically acceptable bile salt under mixing to mixture I to obtain a second mixture (mixture II);
c) adding the at least one water-insoluble vitamin selected from the group consisting of vitamin A, vitamin D, vitamin E, and vitamin K, and pharmaceutically acceptable derivatives thereof, to mixture II under mixing;
d) optionally adding a further amount of mixture II, wherein the ratio of phospholipid to bile salt is identical or different to the mixture prepared in step b; and/or
e) optionally freeze-drying the resulting mixture;

to obtain the pharmaceutical composition.

It was surprisingly found that using the above described method, a pharmaceutical composition may be prepared with a high ratio of water-insoluble vitamin to mixed micelles.

The method of the present disclosure comprises several steps for the preparation of a pharmaceutical composition according to the first aspect of the present disclosure. This method allows for the preparation of a dry or wet pharmaceutical composition with a high ratio of water-insoluble vitamin to mixed micelle. To achieve such high ratio, in a first step, a mixture comprising mixed micelles of at least one phospholipid and at least one bile salt is prepared.

In general, for the preparation of an aqueous mixture of mixed micelles of phospholipid and bile salt, a dispersion of at least one phospholipid in water is prepared in a first step (step a). The phospholipid is dispersed in water until a homogenous dispersion is obtained. This homogenous dispersion of phospholipid in water is also termed as mixture I.

In a second step (step b), at least one bile salt is added to the mixture prepared in step a (mixture I) under mixing to obtain a clear formulation, also termed mixture containing mixed micelles. Said formulation or mixture containing mixed micelles is also referred to as mixture II.

By this method, it is possible to prepare different mixtures II that may contain mixed micelles and water in different amounts, i.e., different concentrations, and also the ratio of bile salt to phospholipid may vary. Both the concentration of the mixed micelles in mixture II as well as the ratio of bile salt to phospholipid may influence the solubility of the water insoluble and liposoluble vitamins in said mixture II.

In a preferred embodiment, the concentration of micelles comprising phospholipid and pharmaceutically acceptable bile salt, also referred to as mixed micelles, in mixture II is preferably above 40 wt.%, further preferably above 45 wt.%, further preferably above 48 wt.%, further preferably at least 50 wt.%. Such concentration is helpful when adding the water-insoluble vitamin to mixture II and reduces the risk of disintegration of the mixed micelles in the process of adding the water-insoluble vitamin. The concentration of micelles comprising phospholipid and pharmaceutically acceptable bile salt is calculated as the total amount of phospholipid and pharmaceutically acceptable bile salt in relation to the total amount of mixture II. It is important to note that the concentration of mixed micelles may be further reduced after the liposoluble vitamins are incorporated by the mixed micelles without resulting in a separation.

After the addition of the pharmaceutically acceptable bile salt, mixture II contains mixed micelles of phospholipid and bile salt, dispersed in water. Said mixture II is the basic mixture for the addition of at least one water-insoluble vitamin. After addition of the at least one water-insoluble vitamin to mixture II, a resulting mixture III is obtained. Further amounts of a mixture II (containing mixed micelles of phospholipid and bile salt) may be added to mixture III. By adding further amounts of mixture II, the resulting composition is stabilized, allowing for the adjustment of the ratio of vitamin to mixed micelle, which may be used to optimize the final pharmaceutical composition for freeze-drying and storing. It is also possible to prepare a further mixture containing mixed micelles at a different ratio of bile salt : phospholipid, thereby adjusting the ratio of bile salt : phospholipid in the final pharmaceutical composition, i.e., a mixture II comprising mixed micelles at a different ratio of bile salt : phospholipid, and at a different concentration . Also this measure may add to the stability of the final pharmaceutical composition.

A mixture containing mixed micelles of at least one phospholipid and at least one bile salt is termed "mixture II", and if additionally water-insoluble vitamins are present, it is referred to as "mixture III". Accordingly, if water-insoluble vitamins are added to mixture II, a mixture III is received. If then further amounts of a mixture II, i.e., a mixture containing mixed micelles of at least one phospholipid and at least one bile salt but no water-insoluble vitamin(s), is added to mixture III, again mixture III is received.

The resulting mixture III may then be lyophilized (freeze-dried) to result in a dry pharmaceutical composition (optional step e). If this optional step of freeze-drying is not performed, the pharmaceutical composition may be administered as such, or a water-soluble vitamin may be further added.

The pharmaceutical composition may additionally contain water-soluble vitamins. In a preferred embodiment of the second aspect of the present disclosure, the method for manufacturing further comprising the steps of

f) providing at least one solution of at least one water-soluble vitamin;

g) mixing the composition of step c or optionally step d and the at least one solution of step f; and

h) optionally freeze-drying the mixture prepared in step g;

to obtain the pharmaceutical composition.

The water-soluble vitamin is preferably provided as aqueous solution and added to mixture III. By adding the water-soluble vitamin as aqueous solution to mixture III (step g), the water-soluble vitamin may be easily integrated into the formulation containing mixed micelles and water-insoluble vitamin(s). The resulting mixture containing water-insoluble and water-soluble vitamins may also be referred to as mixture IV.

The resulting mixture IV may be lyophilized in a similar manner as mixture III (optional step h). The result is a dry pharmaceutical composition with increased stability.

The lyophilization of the pharmaceutical composition may be made according to usual procedures known to the skilled person. The lyophilization may include a step of freezing the composition, such as at -50 °C, followed by drying under reduced pressure (e.g., 250 $\mu$bar at +5 °C), and optionally further followed by further drying at further reduced pressure and elevated temperature (e.g., 10 $\mu$bar at +40 °C).

The resulting dry pharmaceutical composition is preferably storage stable for at least 12 months.

## Examples

In the following, the method for manufacturing of the present disclosure is further illustrated by Examples. These Examples are not considered limiting the scope of the present disclosure, but are intended as being illustrative.

## *Example 1*

The amounts of the components used in the preparation of a vitamin containing pharmaceutical composition according to the present disclosure are provided in Table 1 below.

In step 1, soy lecithin is dispersed in water until a homogeneous dispersion is obtained. Sodium glycocholate is added to the dispersion to obtain a clear formulation containing mixed micelles. In step 2, liposoluble vitamins (vit. A, D, E, K) are added to said formulation under stirring to obtain again a clear formulation. In step 3, a further formulation containing mixed micelles is prepared in the same manner as in step 1 above, and added to the formulation containing the liposoluble vitamins, resulting in a clear formulation. Step 4 includes the addition of hydro-soluble vitamins (vit. C, B1, B2, B3, B5, B6, B7, B9 and B12) dissolved in water to the formulation previously obtained. Subsequently, the pH value is adjusted to pH 6 using sodium hydroxide. The obtained formulation remains clear. In the next step, 42.4 g glycine is added to the formulation and the pH is again adjusted with sodium hydroxide to 6. Afterwards, the remaining clear formulation is sterilized by filtration through a 0.2 $\mu$m polyethersulfone filter into vials, and is subsequently freeze-dried as follows. After freezing the formulation at -50 °C, primary drying is performed at +5 °C under 250 $\mu$bar for at least 40 h, followed by secondary drying at +40 °C at 10 $\mu$bar.

**Table 1** Components of Example 1

| Example 1 | Components | Amount |
|---|---|---|
| Step 1 | Soy lecithin | 1.56 g |
| | Sodium glycocholate | 2.91 g |
| | Water | 4.47 g |
| Step 2 | Vitamin A palmitate | 0.31 g |
| | Vitamin D | 0.85 mg |
| | Vitamin E | 1.54 g |
| | Vitamin K | 25.4 mg |

(continued)

| Example 1 | Components | Amount |
|---|---|---|
| Step 3 | Soy lecithin | 18.6 g |
| | Sodium glycocholate | 21.8 g |
| | Water | 198.8 g |
| Step 4 | Vitamin C | 33.90 g |
| | Vitamin B1 | 1.02 g |
| | Vitamin B2 $NaPO_4$ | 0.78 g |
| | Vitamin B3 | 6.78 g |
| | Vitamin B5 | 2.54 g |
| | Vitamin B6 | 1.02 g |
| | Vitamin B7 | 10.2 mg |
| | Vitamin B9 | 0.10 g |
| | Vitamin B12 | 0.85 mg |
| | Water | 661.5 g |

### Example 2

The experimental procedure is carried out in the same manner as described for Example 1. The amount of the respective components can be taken from Table 2 listed below.

**Table 2** Components of Example 2

| Example 2 | Components | Amount |
|---|---|---|
| Step 1 | Soy lecithin | 1.56 g |
| | Sodium glycocholate | 2.91 g |
| | Water | 4.47 g |
| Step 2 | Vitamin A palmitate | 0.31 g |
| | Vitamin D | 0.85 mg |
| | Vitamin E | 1.54 g |
| | Vitamin K | 25.4 mg |
| Step 3 | Soy lecithin | 13.5 g |
| | Sodium glycocholate | 15.6 g |
| | water | 210 g |

(continued)

| Example 2 | Components | Amount |
|---|---|---|
| Step 4 | Vitamin C | 33.90 g |
| | Vitamin B1 | 1.02 g |
| | Vitamin B2 $NaPO_4$ | 0.78 g |
| | Vitamin B3 | 6.78 g |
| | Vitamin B5 | 2.54 g |
| | Vitamin B6 | 1.02 g |
| | Vitamin B7 | 10.2 mg |
| | Vitamin B9 | 0.10 g |
| | Vitamin B12 | 0.85 mg |
| | Water | 661.5 g |

*Example 3*

The experimental procedure is carried out in the same manner as described for Example 1. The amount of the respective components can be taken from Table 3 listed below.

**Table 3** Components of Example 3

| Example 3 | Components | Amount |
|---|---|---|
| Step 1 | Soy lecithin | 1.56 g |
| | Sodium glycocholate | 2.91 g |
| | Water | 4.47 g |
| Step 2 | Vitamin A palmitate | 0.31 g |
| | Vitamin D | 0.85 mg |
| | Vitamin E | 1.54 g |
| | Vitamin K | 25.4 mg |
| Step 3 | Soy lecithin | 6.0 g |
| | Sodium glycocholate | 6.4 g |
| | Water | 226.8 g |
| Step 4 | Vitamin C | 33.90 g |
| | Vitamin B1 | 1.02 g |
| | Vitamin B2 $NaPO_4$ | 0.78 g |
| | Vitamin B3 | 6.78 g |
| | Vitamin B5 | 2.54 g |
| | Vitamin B6 | 1.02 g |
| | Vitamin B7 | 10.2 mg |
| | Vitamin B9 | 0.10 g |
| | Vitamin B12 | 0.85 mg |
| | Water | 661.5 g |

*Example 4*

The experimental procedure is carried out in the same manner as described for Example 1. The amount of the respective components can be taken from Table 4 listed below.

**Table 4** Components of Example 4

| Example 4 | Components | Amount |
|---|---|---|
| Step 1 | Soy lecithin | 1.56 g |
| | Sodium glycocholate | 2.91 g |
| | Water | 4.47 g |
| Step 2 | Vitamin A palmitate | 0.31 g |
| | Vitamin D | 0.85 mg |
| | Vitamin E | 1.54 g |
| | Vitamin K | 25.4 mg |
| Step 3 | Soy lecithin | 2.2 g |
| | Sodium glycocholate | 1.7 g |
| | Water | 235.3 g |
| Step 4 | Vitamin C | 33.90 g |
| | Vitamin B1 | 1.02 g |
| | Vitamin B2 NaPO$_4$ | 0.78 g |
| | Vitamin B3 | 6.78 g |
| | Vitamin B5 | 2.54 g |
| | Vitamin B6 | 1.02 g |
| | Vitamin B7 | 10.2 mg |
| | Vitamin B9 | 0.10 g |
| | Vitamin B12 | 0.85 mg |
| | Water | 661.5 g |

### Reference Example 5

The following example shows a protocol for producing a composition comprising the same components and amount as the commercially available multivitamin product Viant®. Table 5 summarizes the amount of the respective components used for Reference Example 5.

Soy lecithin is dispersed in water until a homogeneous dispersion is obtained. Sodium glycocholate is added to the solution to obtain a clear formulation. Subsequently, liposoluble vitamins (vit. A, D, E, K) are added to the solution under stirring to obtain again a clear formulation (amounts can be taken from Table 5). Hydro-soluble vitamins (vit. C, B1, B2, B3, B5, B6, B7, B9 and B12) dissolved in water (amounts can be taken from Table 5) are added to the formulation obtained in the previous step. Subsequently, the pH value is adjusted to pH 6 using sodium hydroxide. The obtained formulation remains clear. In the next step, 42.4 g glycine is added to the formulation and the pH is again adjusted with sodium hydroxide to pH 6. The clear formulation is sterilized by filtration through a 0.2 μm polyethersulfone filter into vials, and is subsequently freeze-dried.

**Table 5** Components of Reference Example 5

| Example 5 | Components | Amount |
|---|---|---|
| Mixed micelles (MM) | Soy lecithin | 30.18 g |
| | Sodium glycocholate | 37.09 g |
| | Water | 82.07 g |

(continued)

| Example 5 | Components | Amount |
|---|---|---|
| Liposoluble vitamins | Vitamin A palmitate | 0.31 g |
| | Vitamin D | 0.85 mg |
| | Vitamin E | 1.54 g |
| | Vitamin K | 25.4 mg |
| Hydro-soluble vitamins | Vitamin C | 33.90 g |
| | Vitamin B1 | 1.02 g |
| | Vitamin B2 $NaPO_4$ | 0.78 g |
| | Vitamin B3 | 6.78 g |
| | Vitamin B5 | 2.54 g |
| | Vitamin B6 | 1.02 g |
| | Vitamin B7 | 10.2 mg |
| | Vitamin B9 | 0.10 g |
| | Vitamin B12 | 0.85 mg |
| | Water | 661.5 g |

## Example 6

The stability of the obtained freeze-dried pharmaceutical composition prepared according to Reference Example 5 was tested under different storage conditions over time. The stability was determined in the consistency of transmittance, pH value, reconstitution time of the freeze-dried composition and different parameters of the particle size.

Table 6 displays parameters relevant for the stability of the freeze-dried pharmaceutical composition prepared according to Reference Example 5 after 3, 6 and 12 months at 5 °C and 3 and 6 months at 25 °C storage temperature.

**Table 6** Stability of composition of Reference Example 5

| Time (months) and storage condition | 0; - | 3; 5 °C | 6; 5 °C | 12; 5 °C | 3; 25 °C | 6; 25 °C |
|---|---|---|---|---|---|---|
| % Transmittance, 660 nm | 98 | 99 | 99 | N/A | 99 | 98 |
| pH of reconstituted solution | 6.1 | 6.1 | 6.2 | 6.2 | 6.1 | 6.1 |
| Reconstitution time [s] | 20 | 15 | 15 | 15 | 15 | 15 |
| Size distribution of mixed micelles [nm] | 6 | 6 | 6 | 6 | 6 | 6 |
| Visible particles | none | none | none | none | none | none |
| Subvisible particles $\geq$ 10 $\mu$m (particles/container) | 917 | 685 | 387 | 97 | 440 | 303 |
| Subvisible particles $\geq$ 25 $\mu$m (particles/container) | 0 | 3 | 7 | 13 | 0 | 3 |

## Example 7

The stability of the obtained freeze-dried pharmaceutical composition produced according to Example 1 was tested under different storage conditions over time. The stability was determined in the consistency of transmittance, pH value, reconstitution time of the freeze-dried composition and different parameters for the particle size.

Table 7 displays parameters relevant for the stability of the freeze-dried pharmaceutical composition prepared according to Example 1 after 3, 6 and 12 months at 5 °C and 3 and 6 months at 25 °C storage temperature.

**Table 7** Stability of composition of Example 1

| Time (months) and storage condition | 0; - | 3; 5 °C | 6; 5 °C | 12; 5 °C | 3; 25 °C | 6; 25 °C |
|---|---|---|---|---|---|---|
| % Transmittance, 660 nm | 98 | 100 | 99 | N/A | 100 | 99 |
| pH of reconstituted solution | 6.1 | 6.1 | 6.2 | 6.2 | 6.1 | 6.2 |
| Reconstitution time [s] | 17 | 15 | 15 | 15 | 15 | 15 |
| Size distribution of mixed micelles [nm] | 7 | 7 | 7 | 7 | 7 | 7 |
| Visible particles | none | none | none | none | none | none |
| Subvisible particles $\geq$ 10 $\mu$m (particles/container) | 247 | 367 | 60 | 73 | 173 | 33 |
| Subvisible particles $\geq$ 25 $\mu$m (particles/container) | 3 | 0 | 0 | 7 | 7 | 3 |

### Example 8

The stability of the obtained freeze-dried pharmaceutical composition produced according to Example 3 was tested under different storage conditions over time. The stability was determined at the reconstituted pharmaceutical composition in the consistency of transmittance, pH value, reconstitution time of the freeze-dried composition and different parameters for the particle size. The compositions were reconstituted in 5.7 ml solution for injection.

Table 8 displays parameters relevant for the stability of the freeze-dried pharmaceutical composition prepared according to Example 3 after 3, 6 and 12 months at 5 °C and 3 and 6 months at 25 °C storage temperature, and subsequent reconstitution. The data show that the formulation is stable and clear.

**Table 8** Stability of composition of Example 3

| Time (months) and storage condition | 0; - | 3; 5 °C | 6; 5 °C | 12; 5 °C | 3; 25 °C | 6; 25 °C |
|---|---|---|---|---|---|---|
| % Transmittance, 660 nm | 98 | 100 | 99 | N/A | 99 | 99 |
| pH of reconstituted solution | 6.1 | 6.1 | 6.2 | 6.2 | 6.1 | 6.2 |
| Reconstitution time [s] | 18 | 15 | 15 | 15 | 15 | 15 |
| Size distribution of mixed micelles [nm] | 11 | 10 | 11 | 14 | 14 | 11 |
| Visible particles | none | none | none | none | none | none |
| Subvisible particles $\geq$ 10 $\mu$m (particles/container) | 167 | 253 | 197 | 283 | 230 | 103 |
| Subvisible particles $\geq$ 25 $\mu$m (particles/container) | 10 | 0 | 3 | 27 | 0 | 3 |

### Example 9

Example 9 illustrates the comparison between the freeze-dried pharmaceutical composition resulting from Examples 1 to 4 and Reference Example 5 with regard to the ratio of the amounts of mixed micelles (MM) to the total amount of vitamin. The specific data of the amounts of components per freeze-dried vial for each pharmaceutical component from Examples 1 to 4 and Reference Example 5 can be taken from Table 9. Examples 1 to 4 and Reference Example 5 all contain the same vitamin concentration per vial, whereas the amount of mixed micelles varies. The comparative component is the product shown in Reference Example 5, corresponding to the composition of the commercially available Viant® (B. Braun) multivitamin formulation. Examples 1 to 4 show a reduction in the MM amount compared to Reference Example 5 for which the standard of reduction in MM is set to 0 % as the value to compare. The total amount of the mixed micelles (MM) is calculated as follows:

$$\mathrm{m(MM) = \ m(Soy\ lecithin) + \ m(Sodium\ glycocholate)}$$

Table 9 summarizes the amounts of components in each vial of the resulting freeze-dried pharmaceutical composition of Examples 1 to 4 and Reference Example 5, wherein the amount of lipo- and hydro-soluble vitamins and glycine is identical in all Examples. Solely the amounts of the mixed micelles are varied in this context.

**Table 9** Amounts of mixed micelles in Examples

|  | Ref. Ex. 5 (Viant®) | Ex. 1 | Ex. 2 | Ex. 3 | Ex. 4 |
|---|---|---|---|---|---|
| **Amount of MM relative to Ref. Ex. 5** | 1 (Reference) | 2/3 | 1/2 | 1/4 | 1/8 |
| **Components** | | | | | |
|  | mg/vial | | | | |
| Vitamin A palmitate | 1.82 | | | | |
| Vitamin D | 0.0050 | | | | |
| Vitamin E | 9.11 | | | | |
| Vitamin K | 0.15 | | | | |
| Vitamin C | 200.00 | | | | |
| Vitamin B1 | 6.00 | | | | |
| Vitamin B2 $NaPO_4$ | 4.57 | | | | |
| Vitamin B6 | 6.00 | | | | |
| Vitamin B12 | 0.005 | | | | |
| Vitamin B9 | 0.60 | | | | |
| Vitamin B5 | 15.00 | | | | |
| Vitamin B7 (H) | 0.060 | | | | |
| Vitamin B3 | 40.00 | | | | |
| Glycine | 250.00 | | | | |
| Sodium glycocholate | 218.84 | 145.89 | 109.42 | 54.71 | 27.36 |
| Soy phosphatidylcholine | 178.03 | 118.68 | 89.01 | 44.51 | 22.25 |

Table 10 summarizes the ratios between the amounts of each liposoluble vitamin and mixed micelles (MM) in Examples 1 to 4 compared to Reference Example 5 (Viant®). The amount of liposoluble vitamin is taken in IU (international units) of the respective vitamin for vitamins A, D, and E, and in g for vitamin K. As can be seen, the relative amount of MM is reduced in Examples 1 to 4, compared to Reference Example 5. The table also provides the concentration of MM after reconstitution of the freeze-dried pharmaceutical composition in 5.7 ml solution for injection.

**Table 10** Ratios of liposoluble vitamins to MM

| Ratio | Ref. Ex. 5 (Viant®) | Ex. 1 | Ex. 2 | Ex. 3 | Ex. 4 |
|---|---|---|---|---|---|
| vitamin A / MM [IU/g] | 8.3 | 12.5 | 16.6 | 33.3 | 66.5 |
| vitamin D / MM [IU/g] | 0.50 | 0.76 | 1.01 | 2.02 | 4.03 |
| vitamin E / MM [$10^{-3}$ IU/g] | 25 | 38 | 50 | 101 | 202 |
| vitamin K / MM [$10^3$] | 0.37 | 0.57 | 0.76 | 1.51 | 3.02 |
| **Reduction compared to Ref. Ex. 5 (Viant®)** | 0 % | 33 % | 50 % | 75 % | 88 % |
| **Concentration of MM after reconstitution [mg/ml]** | 69.63 | 46.42 | 34.81 | 17.41 | 8.70 |

*Example 10*

The freeze-dried pharmaceutical compositions of Examples 1 to 4 and Reference Example 5 were tested for their stability after reconstitution to an injectable composition. All Examples were stable for more than 24 h after reconstitution.

Table 11 summarizes the results of the stability testing determined by turbidity (nephelometry) and particle size (dynamic light scattering; DLS) in pharmaceutical compositions reconstituted after freeze-drying of Examples 1 to 5 after 0, 18 and 24 h. The compositions were reconstituted in 5.7 ml solution for injection.

Turbidity measurements (NTU) were performed using a Turbiquant 3000 IR (Merck, Germany) equipped with an infrared light source with a wavelength of 860 nm.

Average size (Zav) was measured with a Zetasizer Nano ZS equipment based on dynamic light scattering principle. Samples were measured undiluted with a detection angle of 175°

**Table 11** Optical properties of pharmaceutical compositions after reconstitution

| Reference | T= 0 | | | T= 18 h | | | T= 24 h | | |
|---|---|---|---|---|---|---|---|---|---|
| | Visual Obs. | NTU | DLS (Zav in nm) | Visual Obs. | NTU | DLS (Zav in nm) | Visual Obs. | NTU | DLS (Zav in nm) |
| Example 5 (Viant®) | clear | 2.2 | 5.8 | clear | 1.8 | 5.9 | clear | 1.7 | 5.7 |
| Example 1 | clear | 1.8 | 6.6 | clear | 1.6 | 6.6 | clear | 1.5 | 6.5 |
| Example 2 | clear | 1.8 | 7.2 | clear | 1.6 | 7.3 | clear | 1.5 | 7.2 |
| Example 3 | clear | 5.4 | 25.0 | clear | 4.8 | 23.6 | clear | 4.6 | 23.3 |
| Example 4 | clear | 7.9 | 21.8 | clear | 9.6 | 23.5 | clear | 10.6 | 24.6 |

### Reference Example 11

Two formulations 11a and 11b were prepared in a manner similar to Reference Example 5, however, without hydro-soluble vitamins, and differing in the concentration of mixed micelles in mixture II when the liposoluble vitamin is added.

In step 1, soy lecithin is dispersed in water until a homogeneous dispersion is obtained (mixture I). Sodium glycocholate is added to the dispersion to obtain a clear formulation (mixture II). In step 2, liposoluble vitamins (vit. A, D, E, K) are added to said formulation under stirring. Table 12 summarizes the amount of the respective components used for Reference Example 11.

**Table 12** Components of Reference Example 11

| Reference Example 11 | Components | Amount | |
|---|---|---|---|
| | | 11a | 11b |
| Step 1 | Soy lecithin | 11.3 g | 12.6 g |
| | Sodium glycocholate | 28.1 g | 31.3 g |
| | Water | 59.2 g | 43.9 g |
| Step 2 | Vitamin A palmitate | 0.23 g | 2.01 g |
| | Vitamin D | 1 mg | 6 mg |
| | Vitamin E | 1.13 g | 10.1 g |
| | Vitamin K | 19 mg | 170 mg |

The resulting formulation is analyzed for its appearance and turbidity before and after dilution with water at a ratio of formulation : water of 1 : 20 (v/v). The results are summarized in Table 13. Turbidity measurements (NTU) were performed using a Turbiquant 3000 IR (Merck, Germany) equipped with an infrared light source with a wavelength of 860 nm.

**Table 13** Optical properties of formulations **11a** and **11b**

| Reference Example 11 | 11a | 11b |
|---|---|---|
| Phospholipid wt. % of mixture I | 16.0 % | 22.3 % |
| Bile salt wt. % of mixture II | 28.5 % | 35.6 % |
| Ratio Bile salt: Phospholipid (molar ratio) | 4 : 1 | 4 : 1 |
| Mixed micelles wt. % of mixture II | 40 % | 50 % |
| | | |

(continued)

| Reference Example 11 | | 11a | 11b |
|---|---|---|---|
| Visual observation | Before dilution | Heterogeneous & turbid | Homogeneous & visually clear |
| | After dilution | Heterogeneous & turbid | Homogeneous & visually clear |
| Turbidity measurements | Before dilution | > 200 NTU | < 3 NTU |
| | After dilution | > 200 NTU | < 3 NTU |

The above given results show that the concentration of micelles comprising phospholipid and pharmaceutically acceptable bile salt, also referred to as mixed micelles, in mixture II when the water-insoluble and liposoluble vitamin is added should preferably be above 40 wt.% to facilitate the solubilization of the liposoluble vitamin to result in a clear and homogenous formulation. While at a concentration of 40 wt.% of mixed micelles, as in formulation **11a,** results in a heterogenous and turbid formulation, a higher concentration of mixed micelles of 50 wt.%, as in formulation **11b,** results in a clear and homogenous formulation. This is even more surprising considering that the amount of liposoluble vitamins is significantly higher for formulation **11b.**

It may additionally be noted that the concentration of mixed micelles may be further reduced once the liposoluble vitamins are incorporated by the mixed micelles without resulting in a separation, shown by a heterogenous and/or turbid formulation. This is exemplified in Examples 1 to 4.

***Reference Example 12***

The amounts of the components used in the preparation of a vitamin containing pharmaceutical composition according to the present disclosure are provided in Table 14 below.

In step 1, soy lecithin is dispersed in water until a homogeneous dispersion is obtained (mixture I). Sodium glycocholate is added to the dispersion to obtain a clear dispersion containing mixed micelles (mixture II). In step 2, liposoluble vitamins (vit. A, D, E, K **(12b)** or vit. E **(12a))** are added to said formulation under stirring.

**Table 14** Components of Reference Example 12

| Reference Example 12 | Components | Amount | |
|---|---|---|---|
| | | **12a** | **12b** |
| Step 1 | Soy lecithin | 26.4 g | 7.37 g |
| | Sodium glycocholate | 22.9 g | 36.5 g |
| | Water | 49.3 g | 43.9 g |
| Step 2 | Vitamin A palmitate | - | 1.81 g |
| | Vitamin D | - | 5 mg |
| | Vitamin E | 1.13 g | 9.11 g |
| | Vitamin K | - | 15 mg |

The resulting formulation is analyzed for its appearance and turbidity before and after dilution with water at a ratio of formulation : water of 1 : 20 (v/v). The results are summarized in Table 15. Turbidity measurements (NTU) were performed using a Turbiquant 3000 IR (Merck, Germany) equipped with an infrared light source with a wavelength of 860 nm.

**Table 15** Optical properties of formulations **12a** and **12b**

| Reference Example 12 | 12a | 12b |
|---|---|---|
| Phospholipid wt. % of mixture I | 34.9 % | 14.4 % |
| Bile salt wt. % of mixture II | 23.2 % | 41.6 % |
| Ratio Bile salt: Phospholipid (molar ratio) | 1.4 : 1 | 7.9 : 1 |
| Mixed micelles wt. % of mixture II | 50 % | 50 % |

(continued)

| Reference Example 12 | | 12a | 12b |
|---|---|---|---|
| | | | |
| Visual observation | Before dilution | Heterogeneous & turbid | Heterogeneous & turbid |
| | After dilution | Heterogeneous & turbid | Heterogeneous & turbid |
| Turbidity measurement | Before dilution | > 200 NTU | > 200 NTU |
| | After dilution | > 200 NTU | > 200 NTU |

The bile salt used had a molar mass of 488 g/mol, and the phospholipid used had a molar mass of 776 g/mol. As can be seen from the results in Table 15, also the ratio of bile salt to phospholipid (molar ratio) after step 1 in the mixture II may help to facilitate the solubilization of the liposoluble vitamin to result in a clear and homogenous formulation in step 2. As well as at low bile salt concentrations **(12a)** or high bile salt concentrations **(12b),** the process does not lead to solubilization of the liposoluble vitamin(s).

**Claims**

1. A pharmaceutical composition comprising mixed micelles, and at least one water-insoluble vitamin selected from the group consisting of vitamin A, vitamin D, vitamin E, and vitamin K, and pharmaceutically acceptable derivatives thereof,

   a) wherein the mixed micelles each comprise at least one phospholipid, and at least one pharmaceutically acceptable bile salt, and
   b) wherein at least one of the following conditions is met:

   i) ratio of vitamin A (total amount in IU) to mixed micelles (total amount in mg) is more than 15 IU/mg, or
   ii) ratio of vitamin D (total amount in IU) to mixed micelles (total amount in mg) is more than 0.9 IU/mg, or
   iii) ratio of vitamin E (total amount in IU) to mixed micelles (total amount in mg) is more than $45 \times 10^{-3}$ IU/mg, or
   iv) ratio of vitamin K (total amount in mg) to mixed micelles (total amount in mg) is more than $0.6 \times 10^{-3}$.

2. The pharmaceutical composition according to claim 1, wherein the pharmaceutical composition further comprises at least one water-soluble vitamin, preferably wherein the at least one water-soluble vitamin is selected from the group consisting of vitamin C, vitamin B1, vitamin B2, vitamin B6, vitamin B12, vitamin B9, vitamin B5, vitamin B7, vitamin B3, and mixtures thereof, further preferably wherein the at least one water-soluble compound is a mixture of vitamin C, vitamin B1, vitamin B2, vitamin B6, vitamin B12, vitamin B9, vitamin B5, vitamin B7, and vitamin B3.

3. The pharmaceutical composition according to any one of claims 1 or 2, wherein the at least one water-insoluble vitamin comprises vitamin A, vitamin D, vitamin E, and vitamin K, or a pharmaceutically acceptable derivative thereof.

4. The pharmaceutical composition according to any one of claims 1 to 3, wherein at least two of the conditions i to iv are met, preferably wherein at least three of the conditions i to iv are met, further preferably wherein all four conditions i to iv are met.

5. The pharmaceutical composition according to any one of claims 1 to 4, wherein the at least one phospholipid is lecithin, preferably phosphatidylcholine, more preferably purified phosphatidylcholine.

6. The pharmaceutical composition according to any one of claims 1 to 5, wherein the pharmaceutically acceptable bile salt is a pharmaceutically acceptable salt of glycocholate, taurocholate or deoxycholate, preferably wherein the pharmaceutically acceptable bile salt is sodium glycocholate.

7. The pharmaceutical composition according to any one of claims 1 to 6, wherein the at least one water-insoluble vitamin comprises vitamin A, preferably in the form of vitamin A palmitate, and vitamin E, and optionally one or more further water-insoluble vitamins, preferably vitamin K and/or vitamin D.

8. The pharmaceutical composition according to any one of claims 1 to 7, wherein the at least one water-insoluble vitamin comprises vitamin A, and wherein the ratio of vitamin A (total amount in IU) to mixed micelles (total amount in mg) is

a) 20 IU/mg or more, preferably 25 IU/mg or more, more preferably 30 IU/mg or more, yet more preferably 40 IU/mg or more, even more preferably 50 IU/mg or more; and/or
b) 100 IU/mg or less, preferably 90 IU/mg or less, more preferably 75 IU/mg or less, yet more preferably 60 IU/mg or less, yet more preferably 50 IU/mg or less, even more preferably 40 IU/mg or less.

9. The pharmaceutical composition according to any one of claims 1 to 7, wherein the at least one water-insoluble vitamin comprises vitamin D, and wherein the ratio of vitamin D (total amount in IU) to mixed micelles (total amount in mg) is

a) 0.95 IU/mg or more, preferably 1.0 IU/mg or more, more preferably 1.3 IU/mg or more, yet more preferably 1.5 IU/mg or more, even more preferably 2.0 IU/mg or more; and/or
b) 10 IU/mg or less, preferably 8 IU/mg or less, more preferably 6 IU/mg or less, yet more preferably 5 IU/mg or less, yet more preferably 4 IU/mg or less, even more preferably 3 IU/mg or less.

10. The pharmaceutical composition according to any one of claims 1 to 7, wherein the at least one water-insoluble vitamin comprises vitamin E, and wherein the ratio of vitamin E (total amount in IU) to mixed micelles (total amount in mg) is

a) $50 \times 10^{-3}$ IU/mg or more, preferably $60 \times 10^{-3}$ IU/mg or more, more preferably $80 \times 10^{-3}$ IU/mg or more, yet more preferably $90 \times 10^{-3}$ IU/mg or more, even more preferably $100 \times 10^{-3}$ IU/mg or more; and/or
b) $400 \times 10^{-3}$ IU/mg or less, preferably $350 \times 10^{-3}$ IU/mg or less, more preferably $300 \times 10^{-3}$ IU/mg or less, yet more preferably $250 \times 10^{-3}$ IU/mg or less, yet more preferably $200 \times 10^{-3}$ IU/mg or less, even more preferably $150 \times 10^{-3}$ IU/mg or less.

11. The pharmaceutical composition according to any one of claims 1 to 7, wherein the at least one water-insoluble vitamin comprises vitamin K, and wherein the ratio of vitamin K (total amount in mg) to mixed micelles (total amount in mg) is

a) $0.65 \times 10^{-3}$ or more, preferably $0.7 \times 10^{-3}$ or more, more preferably $0.75 \times 10^{-3}$ or more, yet more preferably $1.0 \times 10^{-3}$ or more, even more preferably $1.3 \times 10^{-3}$ or more; and/or
b) $10 \times 10^{-3}$ or less, preferably $6 \times 10^{-3}$ or less, more preferably $5 \times 10^{-3}$ or less, yet more preferably $4 \times 10^{-3}$ or less, yet more preferably $3 \times 10^{-3}$ or less, even more preferably $2 \times 10^{-3}$ or less.

12. The pharmaceutical composition according to any one of claims 1 to 11, wherein the pharmaceutical composition is stable for at least 12 months when stored at 30 °C or less, or at 25 °C or less, or at 10 °C or less, or at 5 °C or less, or wherein the pharmaceutical composition, after lyophilization, is stable at least for 24 h after reconstitution in an injectable solution, determined by turbidity and particle size, measured by

a) Nephelometry, with counts of 12 NTU or less, preferably 5 NTU or less, more preferably 3 NTU or less; and/or
b) Dynamic light scattering (DLS), with particles sizes of 25 nm or less, preferably 20 nm or less, more preferably 15 nm or less, yet more preferably 10 nm or less and most preferably 8 nm or less.

13. A method for manufacturing the pharmaceutical composition according to any one of claims 1 to 12 comprising the steps of

a) providing a dispersion of at least one phospholipid in water (mixture I);
b) adding a pharmaceutically acceptable bile salt under mixing to mixture I to obtain a second mixture (mixture II);
c) adding the at least one water-insoluble vitamin selected from the group consisting of vitamin A, vitamin D, vitamin E, and vitamin K, and pharmaceutically acceptable derivatives thereof, to mixture II under mixing;
d) optionally adding a further amount of mixture II, wherein the ratio of phospholipid to bile salt is identical or different to the mixture prepared in step b; and/or
e) optionally freeze-drying the resulting mixture;

to obtain the pharmaceutical composition.

**14.** The method according to claim 13, further comprising the steps of

f) providing at least one solution of at least one water-soluble vitamin;
g) mixing the composition of step c or optionally step d and the at least one solution of step f; and
h) optionally freeze-drying the mixture prepared in step g;

to obtain the pharmaceutical composition.

**15.** The pharmaceutical composition according to any one of claims 1 to 12 for use in a method of treating or preventing a vitamin deficiency in a subject.

Europäisches
Patentamt

European
Patent Office

Office européen
des brevets

# EUROPEAN SEARCH REPORT

**Application Number**

EP 24 17 1976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 110 876 719 B (INST MILITARY MEDICINE ACADEMY MILITARY SCIENCES PLA) 9 August 2022 (2022-08-09) * claims 1-11; examples 1-4 * | 1 | INV. A61K9/107 A61K9/19 A61K31/00 A61K47/28 |
| X | US 5 376 646 A (PITTROF FOLKER [US] ET AL) 27 December 1994 (1994-12-27) * example 10 * | 1,5,6,8, 12 | A61P3/02 A61K9/00 |
| X | WO 2021/121082 A1 (UNIV SICHUAN [CN]) 24 June 2021 (2021-06-24) * example 19 * | 1,3,5,6, 8,12 | |
| X | CN 102 641 276 A (JIANGSU SEMPOLL PHARMACEUTICAL CO LTD) 22 August 2012 (2012-08-22) * claim 9; example 1 * | 1,3,5,8, 12 | |
| X | Baxter: "Cernevit-12 (multivitamins for infusion)", , 1 April 1999 (1999-04-01), XP093212948, Retrieved from the Internet: URL:https://www.accessdata.fda.gov/drugsat fda_docs/label/1999/209241bl.pdf [retrieved on 2024-10-09] * page 1, paragraph 1-5 * * page 2, paragraph 3-5 * | 2,4,7, 13-15 | |

| | TECHNICAL FIELDS SEARCHED (IPC) |
|---|---|
| | A61K A61P |

~~The present search report has been drawn up for all claims~~

| Place of search | Date of completion of the search | Examiner |
|---|---|---|
| The Hague | 9 October 2024 | Schwald, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

\.................................................................................

& : member of the same patent family, corresponding document

EPO FORM 1503 03.82 (P04C01)

Europäisches
Patentamt

European
Patent Office

Office européen
des brevets

Application Number

EP 24 17 1976

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

8(completely); 1-7, 12-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 24 17 1976

The Search Division considers that the present European patent application does not comply with the requirements of unity of invention and relates to several inventions or groups of inventions, namely:

1. claims: 8(completely); 1-7, 12-15(partially)

   A pharmaceutical composition comprising mixed micelles, and at least one water-insoluble vitamin selected from vitamin A, and optionally vitamin D, vitamin E, and vitamin K, and pharmaceutically acceptable derivatives thereof,a) wherein the mixed micelles each comprise at least one phospholipid, and at least one pharmaceutically acceptable bile salt, andb) wherein at least condition (i) is met:i) ratio of vitamin A (total amount in IU) to mixed micelles (total amount in mg) is more than 15 IU/mg, orii) ratio of vitamin D (total amount in IU) to mixed micelles (total amount in mg) is more than 0.9 IU/mg, oriii) ratio of vitamin E (total amount in IU) to mixed micelles (total amount in mg) is more than 45x10-3 1 U/M g, oriv) ratio of vitamin K (total amount in mg) to mixed micelles (total amount in mg) is more than 0.6x10-3 , as well as methods of making it and the composition for use in treating a vitamin deficiency.
   - - -

2. claims: 9(completely); 1-7, 12-15(partially)

   A pharmaceutical composition comprising mixed micelles, and at least one water-insoluble vitamin selected from vitamin D and optionally vitamin A, vitamin E, and vitamin K,and pharmaceutically acceptable derivatives thereof,a) wherein the mixed micelles each comprise at least one phospholipid, and at least one pharmaceutically acceptable bile salt, andb) wherein at least condition (ii) is met:i) ratio of vitamin A (total amount in IU) to mixed micelles (total amount in mg) is more than 15 IU/mg, orii) ratio of vitamin D (total amount in IU) to mixed micelles (total amount in mg) is more than 0.9 IU/mg, oriii) ratio of vitamin E (total amount in IU) to mixed micelles (total amount in mg) is more than 45x10-3 1 U/M g, oriv) ratio of vitamin K (total amount in mg) to mixed micelles (total amount in mg) is more than 0.6x10-3 , as well as methods of making it and the composition for use in treating a vitamin deficiency.
   - - -

3. claims: 10(completely); 1-7, 12-15(partially)

   A pharmaceutical composition comprising mixed micelles, and at least one water-insoluble vitamin selected from vitamin E, and optionally vitamin A, vitamin D, and vitamin K, and pharmaceutically acceptable derivatives thereof,a) wherein the mixed micelles each comprise at least one phospholipid, and at least one pharmaceutically acceptable bile salt, andb) wherein at least the following condition (iii) is met:i) ratio of vitamin A (total amount in IU) to mixed micelles (total amount in mg) is more than 15 IU/mg, orii)

page 1 of 2

25

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

ratio of vitamin D (total amount in IU) to mixed micelles (total amount in mg) is more than 0.9 IU/mg, oriii) ratio of vitamin E (total amount in IU) to mixed micelles (total amount in mg) is more than 45x10-3 1 U/M g, oriv) ratio of vitamin K (total amount in mg) to mixed micelles (total amount in mg) is more than 0.6x10-3 , as well as methods of making it and the composition for use in treating a vitamin deficiency.

- - -

4. claims: 11(completely); 1-7, 12-15(partially)

A pharmaceutical composition comprising mixed micelles, and at least one water-insoluble vitamin selected from vitamin K, and optionally vitamin A, vitamin D, and vitamin E, and pharmaceutically acceptable derivatives thereof,a) wherein the mixed micelles each comprise at least one phospholipid, and at least one pharmaceutically acceptable bile salt, andb) wherein at least the following condition (iv) is met:i) ratio of vitamin A (total amount in IU) to mixed micelles (total amount in mg) is more than 15 IU/mg, orii) ratio of vitamin D (total amount in IU) to mixed micelles (total amount in mg) is more than 0.9 IU/mg, oriii) ratio of vitamin E (total amount in IU) to mixed micelles (total amount in mg) is more than 45x10-3 1 U/M g, oriv) ratio of vitamin K (total amount in mg) to mixed micelles (total amount in mg) is more than 0.6x10-3 , as well as methods of making it and the composition for use in treating a vitamin deficiency.

- - -

## ANNEX TO THE EUROPEAN SEARCH REPORT
## ON EUROPEAN PATENT APPLICATION NO.

EP 24 17 1976

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2024

| Patent document cited in search report | | Publication date | Patent family member(s) | | Publication date |
|---|---|---|---|---|---|
| CN 110876719 | B | 09-08-2022 | NONE | | |
| US 5376646 | A | 27-12-1994 | AT | E124253 T1 | 15-07-1995 |
| | | | AU | 636947 B2 | 13-05-1993 |
| | | | CA | 2033725 A1 | 25-07-1991 |
| | | | DK | 0439042 T3 | 02-10-1995 |
| | | | EP | 0439042 A1 | 31-07-1991 |
| | | | IE | 910235 A1 | 31-07-1991 |
| | | | JP | 3240330 B2 | 17-12-2001 |
| | | | JP | H04210927 A | 03-08-1992 |
| | | | NZ | 236823 A | 26-08-1993 |
| | | | PH | 31460 A | 03-11-1998 |
| | | | US | 5376646 A | 27-12-1994 |
| | | | ZA | 91353 B | 25-09-1991 |
| WO 2021121082 | A1 | 24-06-2021 | CN | 112999356 A | 22-06-2021 |
| | | | WO | 2021121082 A1 | 24-06-2021 |
| CN 102641276 | A | 22-08-2012 | NONE | | |

EPO FORM P0459

For more details about this annex : see Official Journal of the European Patent Office, No. 12/82